# EUROPEAN PATENT APPLICATION

(11) **EP 4 738 380 A1**
(43) Date of publication of application: **06.05.2026**
(21) Application number: 24383190.6
(22) Date of filing: 30.10.2024
(51) Int. Cl.: G16H 30/40, G06F 18/20, G06N 3/02, G06N 20/00, G06T 7/00, G06V 10/82, G06V 40/00, G16H 50/20, G16H 50/30, G16H 50/70

(54) **PREDICTION OF CARDIOVASCULAR EVENT**

(71) Applicant: Fundació Hospital Universitari Vall d'Hebron - Institut de Recerca, 08035 Barcelona (ES); Fundacio per a la Universitat Oberta de Catalunya (UOC), 08018 Barcelona (ES)
(72) Inventor: HERNÁNDEZ PASCUAL, Cristina, 08018 BARCELONA (ES); SIMÓ CANONGE, Rafael, 08173 SANT CUGAT DEL VALLÈS (ES); SIMÓ-SERVAT, Olga, 08197 SANT CUGAT DEL VALLÈS Spain (ES); MASIP, David, 08018 BARCELONA (ES); GONZÁLEZ BARRIADA, Rubén, 24006 León (ES)
(74) Representative: ZBM Patents - Zea, Barlocci & Markvardsen

(57) **Abstract**

A computer-implemented method of prediction of a cardiovascular, CV, event of a patient, based on a retinal image from the patient, is provided. The method comprises extracting a feature data set from the retinal image using a neural network, the neural network being pretrained to output feature data sets based on retinal images; inputting the extracted feature data set to a machine learning model, ML model, the ML model being pretrained to output a CV event indicator, the pretraining being based on the following data: training feature data sets extracted from retinal images; and training CV event indicators, one training CV event indicator per each one of the training feature data sets; and obtaining a CV event indicator of the patient from the ML model. A computer-implemented method of training a deep learning model based on retinal images and CV event indicators wherein the deep learning model comprises a neural network, NN, and a machine learning, ML, model, is provided. A data processing system, a training data processing system and a computer program product are also provided.

## Description

The present disclosure relates to methods and systems used for predicting a risk for a patient to suffer from a cardiovascular event.

### BACKGROUND

Some patients may present cardiovascular event risk compared to other patients. The identification of patients at a relative risk of suffering from a cardiovascular event is a challenge for healthcare professionals.

### SUMMARY

In a first aspect, a computer-implemented method of prediction of a cardiovascular, CV, event of a patient is presented. The method is based on a retinal image from the patient and the method comprises: extracting a feature data set from the retinal image using a neural network, NN, the NN being pretrained to output feature data sets based on retinal images; inputting the extracted feature data set to a machine learning model, ML model; and obtaining a CV event indicator of the patient from the ML model. The ML model is pretrained to output a CV event indicator, the pretraining being based on the following data: training feature data sets extracted from retinal images; and training CV event indicators, one training CV event indicator per each one of the training feature data sets.

### Inference

The above method defines therefore a method for the inference of a risk of a cardiovascular event based on retinal images. The inference is performed by a deep learning model consisting of a combination of the NN and the ML model, wherein the output of the NN is an input for the ML model. The NN is pretrained to output feature data sets based on retinal images. The NN is pretrained to output one feature data set per each input retinal image. In inference mode, the NN outputs or provides feature data sets given sets of retinal images. The ML model is pretrained to output a CV event indicator based on the following data: training feature data sets extracted from retinal images; and training CV event indicators, one training CV event indicator per each one of the training feature data sets. In the present disclosure the feature data sets may be referred to as "samples for the ML model" since such "feature data sets" may be used for training the ML model. In examples the pretraining is based on training feature data sets extracted from the same NN as the neural network used for the inference or prediction of a CV event.

A cardiovascular event may be understood in the present disclosure as any one of heart attack, angina, or stroke.

The patients, in examples, may comprise patients who have suffered from a CV event and patients who have not suffered from a CV event, or control patients.

Extracting a feature data set from the retinal image by a neural network may be performed by a convolutional autoencoder (CAE) or by a variational autoencoder (VAE); such neural networks may be used to extract relevant features from the retinal images in an unsupervised mode. The convolutional autoencoder (CAE) may present an architecture comprising convolutional layers to process the retinal images. CAE may be advantageous for capturing spatial hierarchies and features within the data. The CAE may undergo an unsupervised training, where the neural network learns to encode retinal images into a compressed representation or a latent space and then decode that representation to reconstruct the original retinal image. Through this process, the neural network automatically learns relevant features without the need of labeled data. The variational autoencoder (VAE) may provide a latent space modeled as a probability distribution. The VAE learns to map input retinal images to a continuous latent space, enabling smooth transitions between features. The VAEs may learn to generate new retinal images by sampling from the latent space and decoding the samples. The neural network learns a rich set of features by maximizing the likelihood of reconstructing the input. VAEs may be used for generating new images, through generative modeling, and for extracting meaningful, disentangled features from data in an unsupervised manner. Generative Adversarial Networks (GANs), particularly unsupervised GAN variants, may also learn useful feature representations by generating images.

Neural networks can extract meaningful representations from images without needing explicit labels, making them powerful tools for unsupervised learning.

The neural network of the computer-implemented method may be trained or pretrained in an unsupervised mode to provide feature data sets based on the retinal images.

The neural network may comprise an autoencoder, for example a masked autoencoder or for example an autoencoder based on a Transformer model. A Transformer model may be understood as a deep-learning model that may adopt the mechanism of attention, differentially weighing the significance of each part of the input data. Transformer models may be used in the field of computer vision. A Transformer model does not necessarily process the data in order. The term Transformer is to be understood in line with the common understanding of the person skilled in the art (see e.g., https://en.wikipedia.org/wiki/Transformer (machine_learning_model)). The neural network, in examples, comprises a RETFound model which is a foundation model for retinal images that learns generalizable representations from unlabelled retinal images.

The method of prediction of a CV event further comprises inputting the extracted feature data set to a machine learning model, ML model. The ML model is trained to output a CV event indicator based on training feature data sets extracted by the neural network from retinal images and training CV event indicators, where the training associates one training CV event indicator per each one of the training feature data sets.

The method of prediction of a CV event of a patient further comprises obtaining, and in examples outputting, a CV event indicator where the CV event indicator may comprise a binary value indicating presence or absence of risk of developing a CV event of a patient. The CV event indicator may therefore be represented as a categorical value "risk" or "no risk" or may be an integer 0 or 1. A CV event indicator with a value "1" or "risk" may indicate that the patient may require further investigation to discard or confirm a CV pathology or CV condition.

The methods of the disclosure rely therefore on artificial intelligence (Al) applied on retinal images to predict subjects at relative risk of presenting a cardiovascular event.

The combination of the neural network and the machine learning module may be referred to as a deep learning module or deep learning model.

### Training

In a further aspect, a computer-implemented method of training a deep learning model is described herein. The deep learning model comprises a neural network, NN, and a machine learning, ML, model, wherein the output of the NN is an input for the ML model. The method of training the deep learning model is based on training retinal images and on training CV event indicators. The method of training comprises receiving, by the deep learning model, one or more sets of training retinal images and respective training CV events indicator per retinal image. The receiving may comprise to obtain or to receive from an input interface. The one or more sets of training retinal images may comprise, for example, 1000 images of 224x224 pixels size. The receiving respective training CV event indicators may comprise receiving, from an input interface, a vector of size 1000x1, comprising a number 0 or 1, whose meaning may be 0 for no CV event or 1 for CV event. The wording "respective" refers to a value of training CV event indicator associated to a training retinal image. The inputs to the deep learning model may comprise, for example, a computer-implemented data structure, for example a vector or a matrix or a tensor, for example a tensor of dimensions 1000x224x224x224x3 in an example where the images have 3 RGB channels, having a 1000x224x224x224x3 total data matrix, which corresponds to 1000 images, each 224x224, and with 3 colours each. The computer-implemented data structure comprises training images associated with training CV event indicators.

The method of training further comprises obtaining or extracting training feature data sets from the NN based on the received sets of training retinal images, the NN being pretrained to obtain or output or extract feature data sets based on retinal images.

The method of training further comprises training the ML model to map each output or extracted training feature data set, , to a training CV event indicator by providing, to the ML model, the extracted training feature data sets and the training CV event indicators where each training CV event indicators is associated to a respective training feature data set.

For training the deep learning module, a database of training retinal images may be used; the training images may comprise retinographies or for example non-mydriatic retinographies. The retinal images may comprise a number of retinal images, e.g., between 20.000 and 25.000 retinal images of between 20.000 and 25.000 patients. In examples, some of the patients suffer from diabetic retinopathy (DR). The presence and severity of diabetic retinopathy (DR) may confer a cardiovascular event risk. The training images are used to train the AI module, where the AI module implements a deep-learning model, where the deep learning model comprises the combination of the NN and the ML model. An AI module implementing the deep learning model allows identifying patients at risk of presenting a cardiovascular event, e.g., at risk of developing a cardiovascular disease.

The computer-implemented method for training is based on training data from patients who have suffered from a CV event and patients who have not suffered from a CV event, also referred to as control patients.

In examples the method of training further comprises training the NN to extract feature data sets based on sets of training retinal images by providing, to the NN, the set of training retinal images.

In examples the method of training further comprises further comprises eliminating, once the autoencoder has been trained, the reconstruction section of the autoencoder. In examples the compression part is kept. In examples the compression part provides 1024 features that condense the relevant information of the retinographies. A classifier based on the ML model configured to provide a CV event indicator is fed with the 1024 features.

In a further aspect, a data processing system or computing device is defined herein. The data processing system or computing device comprises a processor configured to carry out the steps of a computer-implemented method of prediction of a cardiovascular, CV, event of a patient, based on a retinal image from the patient according to the methods defined herein. In examples the data processing system comprises a first interface to obtain retinal images; and a second interface to output a CV event indicator.

In a further aspect, a training data processing system is defined, comprising a processor configured carry out the steps of a computer-implemented method of training a deep learning model according to the methods disclosed herein.

In a further aspect, a computer program product is defined, comprising program instructions for causing a computing system to perform a method according to any one of the computer-implemented methods of the disclosure.

The computer program may be in the form of source code, object code, a code intermediate source and object code such as in partially compiled form, or in any other form suitable for use in the implementation of the processes. The carrier may be any entity or device capable of carrying the computer program.

For example, the carrier may comprise a storage medium, such as a ROM, for example a CD ROM or a semiconductor ROM, or a magnetic recording medium, for example a hard disk. Further, the carrier may be a transmissible carrier such as an electrical or optical signal, which may be conveyed via electrical or optical cable or by radio or other means.

When the computer program is embodied in a signal that may be conveyed directly by a cable or other device or means, the carrier may be constituted by such cable or other device or means.

Alternatively, the carrier may be an integrated circuit in which the computer program is embedded, the integrated circuit being adapted for performing, or for use in the performance of, the relevant methods.

### BRIEF DESCRIPTION OF THE DRAWINGS

Non-limiting examples of the present disclosure will be described in the following, with reference to the appended drawings, in which:
Figure 1 shows a representation of a system 100 comprising a deep learning module.
Figure 2 shows a representation of a system 200 comprising a trained deep learning module.
Figure 3 shows a block diagram of steps carried out to implement the computer-implemented method 300 of training a deep learning model based on retinal images according to the present disclosure.
Figure 4 shows a block diagram of steps carried out to implement the computer-implemented method of prediction of a cardiovascular event, CV event, of a patient, based on a retinal image from the patient according to the present disclosure
Figure 5 is a block diagram of an example computing environment 500 including a data processing system or computing device 510 configured to implement aspects of computer-implemented methods and computer-executable program instructions (or code) according to the present disclosure.

### DETAILED DESCRIPTION

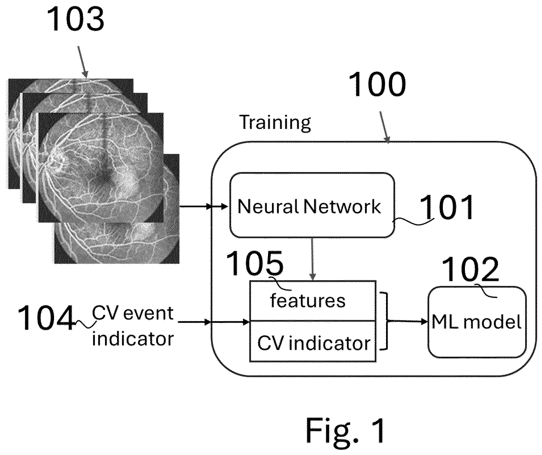

Figure 1 shows a representation of a system 100 comprising a deep learning module. The system is configured to implement the method of training according to the present disclosure. The system comprises a neural network 101. The output of the neural network is an input to an ML model 102. The system 100 is configured to receive a set 103 of training images, the images being retinal images, and training CV event indicators 104. The system 100 is configured to be trained to output a CV event indicator based on a retinal image, as described in the present disclosure. The neural network, NN, 101 is configured to receive the set 103 of training retinal images and to obtain sets of extracted training features 105. The ML model 102 is configured to receive the -extracted- training features 105 and the training CV event indicators 104.

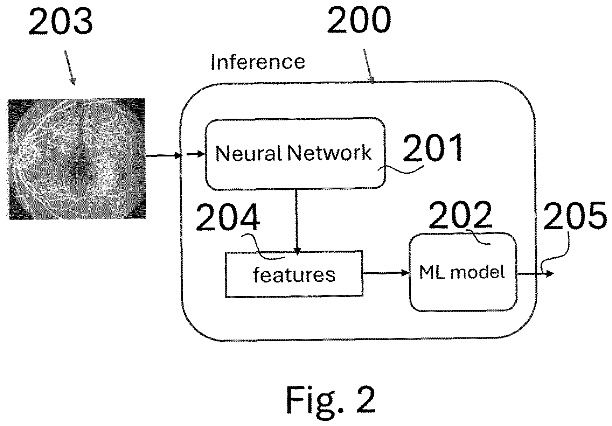

Figure 2 shows a representation of a system 200 comprising a trained deep learning module. The system 200 comprises a trained neural network 201. The output of the neural network 201 is an input to a trained ML model 202. The output of the neural network 201 is a set of features 204. The system 200 is configured to receive a retinal image 203 and to obtain, as output, a CV event indicator 205. The system 200 is configured to infer a CV event indicator based on the retinal image 203, as described in the present disclosure. The neural network, NN, 201 is configured to receive retinal image 203 and to obtain the inferred features 204. The ML model 202 is configured to receive the inferred features 204 and to obtain or output the inferred CV event indicator 205.

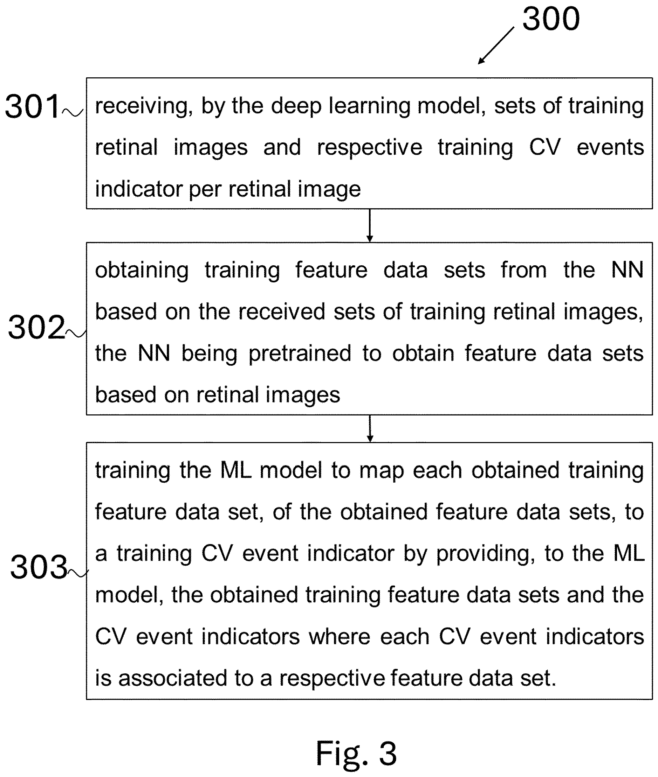

Figure 3 shows a block diagram of steps carried out to implement the computer-implemented method 300, or method 300, of training a deep learning model based on retinal images according to the present disclosure. The method of training may be implemented by the system 100 of figure 1. The method of training comprises, in block 301, receiving, by the deep learning model, sets of training retinal images and respective training CV events indicator per retinal image; the method 300 of training comprises, in block 302, obtaining training feature data sets from the NN based on the received sets of training retinal images, the NN being pretrained to obtain feature data sets based on retinal images; and the method 300 of training comprises, in block 303, training the ML model to map each obtained training feature data set, of the obtained feature data sets, to a training CV event indicator by providing, to the ML model, the obtained training feature data sets and the CV event indicators where each CV event indicators is associated to a respective feature data set.

In examples, the method of training comprises receiving a data set comprising a number, for example between 1400 and 2000 retinal fundus images, RFI, the RFI comprising a first range, e.g., between 60% and 70%, of RFI acquired from patients that had a cardiovascular (CV) event, for example up to N years before the training, N for example being 4 or 5 or 6 and, and the RFI further comprising a second range, e.g., between 30% and 40%, of RFI acquired from patients that never suffered from a CV event, which may be referred to as "control group". In an example, 1483 RFls are acquired, of which 970 RFIs come from patients with CV event and 513 RFI from a control group. In examples, the method of training comprises receiving a data set comprising 254 RFI, of which 165 with CV event and 89 RFI of control.

The patients may comprise patients who have suffered from a disease, such as diabetic patients, for example patients with type 2 diabetes, and/or patients with diabetic retinopathy and/or patients and/or patients suffering from Alzheimer, and/or patients suffering from no disease. In examples, the retinal images from which a CV event indicator is to be inferred comprise retinal images from patients with type 2 diabetes and/or the retinal images comprise retinal images from patients with diabetic retinopathy and/or the retinal images comprise retinal images from patients suffering from Alzheimer, and/or the retinal images comprise retinal images from patients suffering from no disease.

In examples, the training retinal images comprise training retinal images from patients with type 2 diabetes and/or the training retinal images comprise retinal images from patients with diabetic retinopathy and/or the training retinal images comprise retinal images from patients with diabetic retinopathy, and/or the training retinal images comprise retinal images from patients suffering from no disease.

In examples, each training sample of the deep learning model consists of a retinal image and a discrete label, the discrete label being either "CV event" or "no CV event".

In examples the performance of the deep learning model may be analyzed in terms of the accuracy to predict a CV event with respect to two population groups, where a first group may comprise patients with DR (Diabetic Retinopathy) and a second group may comprise patients not diagnosed DR, for example 178 patients.

In examples, the neural network is pretrained to output one set of extracted features based on a retinal image, where one set may comprise one or more features which may be meaningful and relevant features, and which may correspond to different aspects of the retinal structure or potential abnormalities. The features usually represent critical information for medical diagnosis and disease detection. The features may be referred to as "retinal fingerprint", where a retinal fingerprint may refer to a pattern of features within the retina of a patients' eye that may be used to identify or predict risk of developing a CV event. The term "retinal fingerprint" may be associated with biometric identification systems that use retinal scans for medical purposes. The neural network may be pretrained to extract various biologically relevant features from retinal images, which may be relevant for detecting retinal features, leading to better diagnostic tools and insights for clinicians. The set or sets of extracted features may comprise one or more of the following:
- vascular structure or blood vessels, where the neural network may be pretrained to detect and encode the pattern and branching of retinal blood vessels;
- optic disc and cup: the size, shape, and boundary of an area where the optic nerve connects to the retina;
- cup-to-disc ratio: the proportion of the optic cup relative to the optic disc;
- macula: The neural network may be pretrained to identify the macula, the central area of the retina responsible for high-acuity vision;
- fovea: The central part of the macula, the fovea, is relevant for sharp vision;
- lesions or abnormalities: lesions that appear as bright spots, dark spots, or abnormal features in retinal images, such as exudates, hemorrhages, and microaneurysms;
- pigmentation: abnormal pigmentation may be relevant;
- retinal asymmetry: neural networks may extract relevant features.

The above features may be relevant to identify or predict the risk of developing a CV event in the methods of the present disclosure.

In examples, the machine learning model may be of different types. In some examples, given images of retinographies from patients suffering from diabetic retinopathy, using an ML model of the type of Logistic regression provided with a success rate of 83.88% in a confidence interval, Cl, of 3.2%. In some examples, using a ML model of the type K-Nearest Neighbors, KNN, provided with a success rate of 86.17% in a CI of 1.66%. In some examples, using a ML model of the type of Support Vector Machine, SVM, provided with a success rate of 85.25% with CI 1.31%. In some examples, using a ML model of the type of Random Forest provided with a success rate of 83.41 % in a CI of 0.6%. In some examples, using a ML model of the type of Multi-Layer Perceptron , MLP, provided with a success rate of 86.17% in a CI of 2.15%.

In examples, patients suffer from diabetes, also referred to as patients who have diabetes mellitus (DM), e.g., type 1 or type 2 diabetes, or DM patients. DM patients may have a higher burden of atherosclerosis with a higher prevalence of multivessel coronary artery disease (CAD) and myocardial infarction compared to non-DM patients. Once a cardiovascular event or cardiovascular (CV) disease (D) appears, DM patients may have a worse prognosis compared to non-DM patients. A higher CVD risk is not homogeneous in DM patients: some DM patients may never experience CV complications and, for instance, current guidelines consistently advise against the routine use of prophylactic aspirin in DM patients. The methods of the example allow for an early identification of patients with diabetes at risk of developing CV event or CVD risk.

In examples the computer-implemented method of training comprises training data augmentation, by comprising:
- transforming retinal images of an initial set of retinal images by adding occlusions at different points of the retinal images;
- combining the initial set of retinal images with the transformed retinal images; and
- providing thereby at least one set of training retinal images, the at least one set of training retinal images comprising an increased number of retinal images compared to the initial set of retinal images.

In examples the NN is an autoencoder and the training of the autoencoder comprises:
- mapping each training retinal image into a respective training feature data set by using an encoding section of the autoencoder; and
- reconstructing each mapped training retinal image from the corresponding mapped training feature data set by using a reconstruction section of the autoencoder;
- wherein each one of the training feature data sets has a lower dimension than a first initial dimension or initial dimension of the respective retinal image;
- and wherein the mapping and the reconstructing are performed using a loss function.

In examples the loss functions may comprise one of several types of loss functions depending on the type of autoencoder and the desired characteristics of the learned features. The loss function may comprise reconstruction loss, where the goal of the autoencoder is to minimize the difference between the input image and the reconstructed output image. Reconstruction loss may be typically based on a comparison between the input and output images. Reconstruction loss may comprise Mean Squared Error (MSE) or Mean Absolute Error (MAE), or binary cross-entropy loss (BCE). The loss function may alternatively comprise perceptual loss, or kl divergence, or regularization losses (L2 Regularization (Weight Decay), or sparse regularization (L1 Loss on Latent Space)), or adversarial loss or contrastive loss.

In examples, the method of training may also comprise receiving clinical and sociodemographic data to take benefit from the complementary prediction capabilities of image and textual qualitative data.

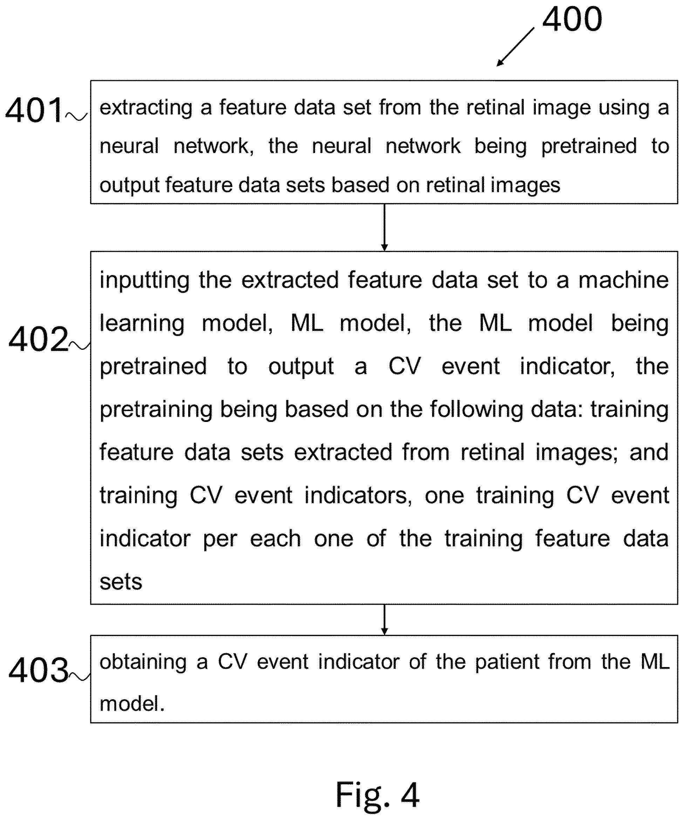

Figure 4 shows a block diagram of steps carried out to implement the computer-implemented method of prediction of a cardiovascular, CV, event of a patient, based on a retinal image from the patient according to the present disclosure. The method of prediction may be implemented by the system 200 of figure 2. The method of prediction comprises, in block 401, extracting a feature data set from the retinal image using a neural network, the neural network being pretrained to output feature data sets based on retinal images. The method of prediction comprises, in block 402, inputting the extracted feature data set to a machine learning model, ML model, the ML model being pretrained to output a CV event indicator, the pretraining being based on the following data: training feature data sets extracted from retinal images; and training CV event indicators, one training CV event indicator per each one of the training feature data sets. The method of prediction comprises, in block 403, obtaining a CV event indicator of the patient from the ML model.

In examples the method of prediction of a CV event allows providing a computer vision method that receives as an input a retinography and returns a label with a likelihood of suffering a CV in the near future. The implementation is based on applying a feature extraction on each retinography using a Deep Learning foundation model, for example a RetFound. The RetFound model is based on training a large Deep Autoencoder with millions of retinographies in an unsupervised way. The architecture uses masked autoencoders with Transformers technology, with an encoder that projects each retinography on a latent space, and a decoder that uncompresses the features of the latent spaces back to the original image. The model is pretrained to reconstruct retinographies from different patients, such as patients with Glaucoma, DR, or cardiovascular diseases. The encoder is applied from the RetFound model to take benefit of the pretrained model, and discard the decoder outputs. The latent representation from each retinography is collected to train a final classifier. In an example, the projection on the latent space is used for samples or images from specific 248 patients, 87 of them without CV event, i.e., control patients, and 161 with CV event, with a total of 1447 retinographies from which 501 retinographies are control retinographies i.e., without CV event, and 946 with CV event. In an example, there are 36 patients with DR and 218 images - several images per patient: in his examples a mean of about 6 images per patient are used-. A group of machine learning classifiers is trained on the prediction of the CV risk or CV event indictor. In examples the validation is performed following a K-Fold cross-validation protocol.

The methods and systems described provide a two-fold improvement: with respect to the medical practice, they provide a tool to preventively mitigate the effects of CV diseases in DR patients by identifying them prior to the event and providing them early treatments. With respect to the state-of-art, preliminary studies on a small sample size data set shows a statistically significant improvement on the prediction of future CV events on patients suffering RD.

The methods and systems defined herein may comprise employing the CV event indicator to identify patients at a risk of presenting a cardiovascular event, allowing to identify a subset of patients in which the screening for cardiovascular disease, CVD, may be prioritized and the control of risk factors may be optimized.

As seen in the present description, artificial intelligence allows identifying by the retina image taken at the moment of a screening of DR those diabetic patients that are at very high risk of presenting a cardiovascular event. In this subgroup of patients, optimizing cardiovascular risk factors and even performing specific tests to detect asymptomatic cardiovascular disease should be prioritized. The following groups will benefit from the methods and systems presented herein: Patients living with diabetes, Health care providers, Insurance companies, general practitioners, GPs, and all professionals attending people with diabetes. Patients with DR have higher cardiovascular risk than those without DR. The risk that confers the presence of DR may be higher than classical risk factors such as hypertension or hypercholesterolemia. The methods and systems presented herein exploit such evidence. In examples, an algorithm is created based on Al-deep learning on the images obtained by non-mydriatic retinography aimed at identifying patients at the highest risk of presenting a cardiovascular event, allowing to identify a subset of diabetic population in which the screening for CVD may be prioritized and the control of risk factors may be optimized, thus reducing the diabetes-associated economic burden. Making primary care professionals aware of a risk of presenting cardiovascular events, especially if the tool has robust results, as expected, might change clinical practice. Focusing the efforts in a high-risk subgroup of patients will result in a cost-efficiency strategy. The methods and systems presented herein may be tested in patients with different pathologies and may trigger an interest of companies to use retinal imaging as a key element for predicting CVD and cardiovascular outcomes in general.

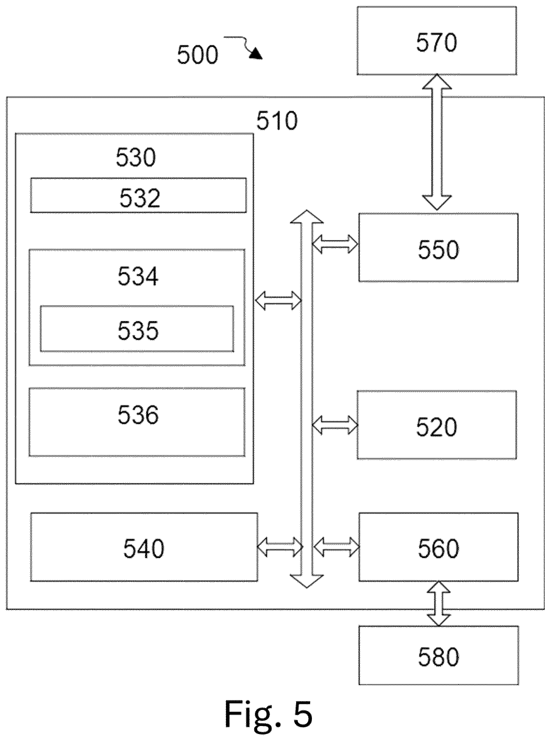

Figure 5 is a block diagram of an example computing environment 500 including a data processing system or computing device 510 configured to implement aspects of computer-implemented methods and computer-executable program instructions (or code) according to the present disclosure. For example, the computing device 510, or portions thereof, is configured to execute instructions to initiate, perform, or control one or more operations described with reference to figures 3 and 4.

The computing device 510 includes one or more processors 520. The processor(s) 520 are configured to communicate with system memory 530, one or more storage devices 540, one or more input/output interfaces 550, one or more communications interfaces 560, or any combination thereof. The system memory 530 includes volatile memory devices (e.g., random access memory (RAM) devices), nonvolatile memory devices (e.g., read-only memory (ROM) devices, programmable read-only memory, and flash memory), or both. The system memory 530 stores an operating system 532, which can include a basic input/output system for booting the computing device 510 as well as a full operating system to enable the computing device 510 to interact with users, other programs, and other devices. The system memory 530 stores program data 536 (e.g., system data). The system memory 530 includes one or more applications 534 (e.g., sets of instructions) executable by the processor(s) 520. As an example, the one or more applications 534 include instructions executable by the processor(s) 520 to operations described with reference to figures 3 and 4.

In examples, the system memory 530 includes a non-transitory, computer readable medium storing instructions 535 that, when executed by the processor(s) 520, cause the processor(s) 520 to initiate, perform, or control operations to predict a risk of developing a CV event. The operations include obtaining a CV event indicator based on retinal images as described in the various examples of the disclosure.

The one or more storage devices 540 include nonvolatile storage devices, such as magnetic disks, optical disks, or flash memory devices. In a particular example, the storage devices 540 include both removable and non-removable memory devices. The storage devices 540 are configured to store an operating system, images of operating systems, applications (e.g., one or more of the applications 534), and program data (e.g., the program data 536). In examples, the system memory 530, the storage devices 540, or both, include tangible (i.e., non-transitory) computer-readable media. In examples, one or more of the storage devices 540 are external to the computing device 510.

The one or more input/output interfaces 550 enable the computing device 510 to communicate with one or more input/output devices 570 to facilitate user interaction. For example, the one or more input/output interfaces 550 can include a display interface, an input interface, or both. For example, the input/output interface 550 is adapted to receive input from a user, to receive input from another computing device, or a combination thereof, where the input comprises retinal images. In some implementations, the input/output interface 550 conforms to one or more standard interface protocols, including serial interfaces (e.g., universal serial bus (USB) interfaces or Institute of Electrical and Electronics Engineers (IEEE) interface standards), parallel interfaces, display adapters, audio adapters, or custom interfaces. In some implementations, the input/output device 570 includes one or more user interface devices and displays, including some combination of buttons, keyboards, pointing devices, displays, speakers, microphones, touch screens, and other devices.

The processor(s) 520 are configured to communicate with devices or controllers 580 via the one or more communications interfaces 560. For example, the one or more communications interfaces 560 can include a network interface. The devices or controllers 580 can include, for example, devices that measure data associated with an aircraft, network devices, client devices, servers, cloud-based devices, one or more other devices, or any combination thereof.

In some implementations, a non-transitory, computer readable medium stores instructions that, when executed by one or more processors, cause the one or more processors to initiate, perform, or control operations to perform part or all of the functionality described above. For example, the instructions can be executable to implement one or more of the operations or methods of figures 3 and 4. In some implementations, part or all of one or more of the operations or methods of figures 3 and 4 can be implemented by one or more processors (e.g., one or more central processing units (CPUs), one or more graphics processing units (GPUs), one or more digital signal processors (DSPs), one or more field-programmable gate arrays (FPGAs), or one or more application-specific integrated circuits (ASICs)) executing instructions, by dedicated hardware circuitry, or any combination thereof.

## Claims

1. A computer-implemented method of prediction of a cardiovascular, CV event of a patient, based on a retinal image from the patient, the method comprising:
- extracting a feature data set from the retinal image using a neural network, the neural network being pretrained to output feature data sets based on retinal images;
- inputting the extracted feature data set to a machine learning model, ML model, the ML model being pretrained to output a CV event indicator, the pretraining being based on the following data:
- training feature data sets extracted from retinal images; and
- training CV event indicators, one training CV event indicator per each one of the training feature data sets; and
- obtaining a CV event indicator of the patient from the ML model.

2. The computer-implemented method of prediction of a CV event of claim 1, wherein the training feature data sets are extracted using the neural network pretrained to output feature data sets based on retinal images.

3. The computer-implemented method of prediction of a CV event of any of claims 1 to 2, wherein the neural network comprises an autoencoder.

4. The computer-implemented method of prediction of a CV event of claim 3 wherein the autoencoder is an autoencoder based on the Transformer model.

5. The computer-implemented method of prediction of a CV event of any one of claims 1 to 4 wherein the machine learning model comprises one of:
- Logistic regression; or
- K-Nearest Neighbors, KNN; or
- Support Vector Machine, SVM; or
- Random Forest; or
- Multi-Layer Perceptron , MLP; or
- AdaBoost; or
- Bayesian classifiers ; or
- Decision Trees.

6. The computer-implemented method of prediction of a CV event of any one of claims 1 to 5 wherein the CV event indicator comprises a binary value indicating presence or absence of risk of developing a CV event of a patient.

7. A computer-implemented method of training a deep learning model based on retinal images and cardiovascular, CV, event indicators, wherein the deep learning model comprises a neural network, NN, and a machine learning, ML, model; the method comprising:
- receiving, by the deep learning model, one or more sets of training retinal images and respective training CV events indicator per retinal image;
- obtaining training feature data sets from the NN based on the received one or more sets of training retinal images, the NN being pretrained to obtain feature data sets based on retinal images; and
- training the ML model to map each obtained training feature data set, of the obtained training feature data sets, to a training CV event indicator by providing, to the ML model, the obtained training feature data sets and the respective training CV event indicators where each CV event indicators is associated to a respective feature data set.

8. The computer-implemented method according to claim 7 of training further comprising training the NN to extract feature data sets based on at least part of the sets of training retinal images by providing, to the NN, the sets of training retinal images.

9. The computer-implemented method of training according to claim 7 or 8 wherein the neural network is an autoencoder and the training of the autoencoder comprises:
- mapping each retinal image into a respective feature data set by using an encoding section of the autoencoder; and
- reconstructing each mapped retinal image from the respective mapped feature data set by using a reconstruction section of the autoencoder;
- wherein each retinal image has an initial dimension;
- wherein each one of the feature data sets has a lower dimension than the initial dimension; and
- wherein the mapping and the reconstructing are performed using a loss function.

10. The computer-implemented method of training according to any one of claims 7 to 9 further comprising:
- transforming retinal images of an initial set of retinal images by adding occlusions at different points of the retinal images;
- combining the initial set of retinal images with the transformed retinal images; and
- providing thereby at least one set of the one or more sets of training retinal images, the at least one set of retinal images comprising an increased number of retinal images compared to the initial set of retinal images.

11. The computer-implemented method according to claim 9 of training further comprising: eliminating, once the autoencoder has been trained, the reconstruction section of the autoencoder.

12. A data processing system, the data processing system comprising a processor configured to carry out the steps of a computer-implemented method according to any one of claims 1 to 6 of prediction of a cardiovascular, CV, event of a patient, based on a retinal image from the patient.

13. The data processing system of claim 12 further comprising:
- a first interface to obtain retinal images; and
- a second interface to output a CV event indicator.

14. A training data processing system comprising a processor configured to carry out the steps of a computer-implemented method of training a deep learning model according to any one of claims 7 to 11.

15. A computer program product comprising program instructions for causing a computing system to perform a method according to any one of claims 1 to 6 or 7 to 11.
